# EUROPEAN PATENT APPLICATION

(11) **EP 3 127 583 A1**
(43) Date of publication of application: **08.02.2017**
(21) Application number: 16186307.1
(22) Date of filing: 24.06.2013
(51) Int. Cl.: A61Q 11/00, A61K 8/24

(54) **A METHOD OF DEMONSTRATING STAIN-PROOF EFFICACY OF AN ORAL CARE COMPOSITION**

(62) Divisional of application: 13883847.9
(71) Applicant: The Procter & Gamble Company, Cincinnati, OH 45202 (US)
(72) Inventor: Strand, Ross, 138547 Singapore (SG); Li, Yujun, Beijing, Beijing 101312 (CN); Zhang, Yiqun, Beijing, Beijing 101312 (CN); Li, Xiaoxiao, Beijing, Beijing 101312 (CN)
(74) Representative: Joos, Uta Susanne

(57) **Abstract**

The present invention provides a method of demonstrating stain-proof efficacy of an oral care composition, comprising the steps:
(a) sticking hydroxyapatite powder uniformly onto a substrate;
(b) immersing the substrate sticked with hydroxyapatite powder into a slurry or solution of the oral care composition;
(c) drying the immersed substrate; and
(d) contacting the dried substrate with a slurry or solution of stain to observe stain's behavior.

## Description

### FIELD OF THE INVENTION

The present invention relates to a method of preventing a stain from depositing on a tooth surface or other oral surfaces. The present invention further relates to a method of demonstrating stain-proof efficacy of an oral care composition, a demonstration product comprising a substrate coated with hydroxyapatite as well as the use of the substrate coated with hydroxyapatit.

### BACKGROUND OF THE INVENTION

Oral care products such as toothpastes and mouthwashes are routinely used by consumers as part of their oral care hygiene regimens. Oral care products are formulated to provide both therapeutic and cosmetic hygiene benefits. Therapeutic benefits include caries prevention which is typically delivered through the use of various fluoride salts; gingivitis prevention by the use of antimicrobial agents such as triclosan, cetylpyridinium chloride, stannous fluoride, zinc citrate or essential oils; and hypersensitivity control through the use of ingredients such as strontium chloride, stannous fluoride or potassium nitrate. Cosmetic benefits include control of plaque and calculus formation, removal and prevention of tooth stain, tooth whitening, breath freshening, and overall improvements in mouth feel impression which can be broadly characterized as mouth feel aesthetics. For example, agents such as pyrophosphate salts have been used as antitartar agents and polymeric agents such as condensed phosphorylated polymers, polyphosphonates, and carboxylated polymers have been used in oral care compositions to provide benefits including tooth surface conditioning and control of tartar, staining and astringency.

The surfaces in the oral cavity are constantly exposed to endogenous and exogenous factors including plaque bacteria and acid, chemicals such as surfactants, harsh abrasives and oral care actives, dietary acids and staining agents that significantly impact the health and appearance of the oral cavity. While oral hygiene products are generally useful for cleaning and protecting the oral cavity from these factors, short term exposure and rapid clearance of actives from the oral cavity minimizes the longer term effects of actives contained in oral hygiene products. In addition, salivary composition, bacteria, and soft tissue abrasion further impacts retention of actives in the oral cavity. The above problems affect many consumers and there continues to be a search for more effective treatment and prevention options.

The present invention is based on the discovery that compositions comprising agents that effectively modify teeth and other oral cavity surfaces to be hydrophobic provide protection against many undesirable conditions including bacterial adhesion, plaque, tartar, caries, erosion, sensitivity, tooth staining and desquamation. The present compositions also provide appearance and textural benefits including shine, smoothness and clean tooth feel.

### SUMMARY OF THE INVENTION

The present invention provides a method of demonstrating stain-proof efficacy on an oral care composition comprising the steps:
(a) sticking hydroxyapatite powder uniformly onto a substrate;
(b) immersing the substrate sticked with hydroxyapatite powder into a slurry or solution of the oral care composition;
(c) drying the immersed substrate; and
(d) contacting the dried substrate with a slurry or solution of stain to observe stain's behavior.

The present invention further provides a demonstration product comprising a substrate coated with hydroxyapatite and the use of the substrate coated with hydroxyapatite for demonstration stain-proof efficacy or for preparing the demonstration product.

Therefore a stain-proof oral care composition is provided comprising:
(a) a zinc source selected from the group consisting of zinc citrate, zinc citrate dehydrate, and mixtures thereof; and
(b) a surface-active organophosphate compound, wherein the surface-active organophosphate compound is represented by the following general structure: 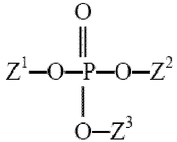
wherein Z¹, Z², or Z³ may be identical or different, wherein at least one of Z¹, Z² , and Z³ is an organic moiety selected from the group consisting of C1-C22 linear or branched alkyls and alkenyls, optionally substituted by one or more phosphate groups; alkoxylated C1-C22 alkyls and alkenyls; saccharides; polysaccharides; polyols; and polyethers.

Therefore, an oral care composition comprising selected zinc source and selected surface active organophosphate compounds and use of these compositions for treating and modifying teeth and other oral cavity surfaces is provided. When applied to oral cavity surfaces, the present composition forms a substantially hydrophobic coating of the surface active organophosphate compound(s) on the treated surface. The organophosphate compound deposits on teeth and mucosal surfaces via bonds or linkages formed between the phosphate groups of the compound and cationic sites on the target surfaces. The treated surface is provided with increased hydrophobic character, which then imparts multiple end use benefits to that surface including ease of cleaning; increased retention of actives such as fluoride on teeth; and surface protection benefits, in particular improved resistance of teeth to bacterial or biofilm adhesion, erosive demineralization or dissolution, sensitivity and staining and prevention of tooth damage from subsequent exposure to erosive chemicals such as acidic foods and beverages. Appearance and textural benefits including smoothness, shine, glossiness, and clean tooth feel are also provided. The organophosphate may be used in combination with one or more other hydrophobic material to further increase hydrophobicity and/or improve functionality of the coating deposited on the surface.

These and other features, aspects, and advantages of the present invention will become evident to those skilled in the art from the detailed description which follows.

### BRIEF DESCRIPTION OF THE DRAWINGS

While the specification concludes with claims particularly defining and distinctly claiming the invention, it is believed that the invention will be better understood from the following description of the accompanying figures. In the accompanying figures,

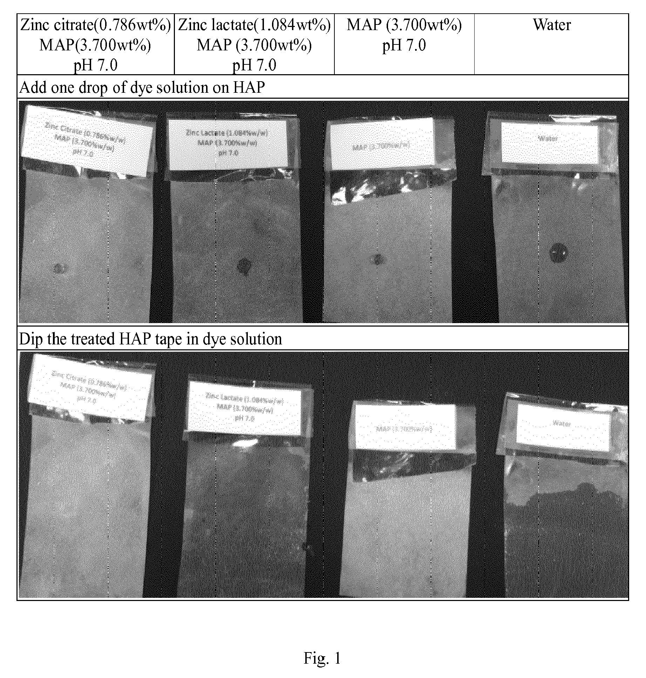

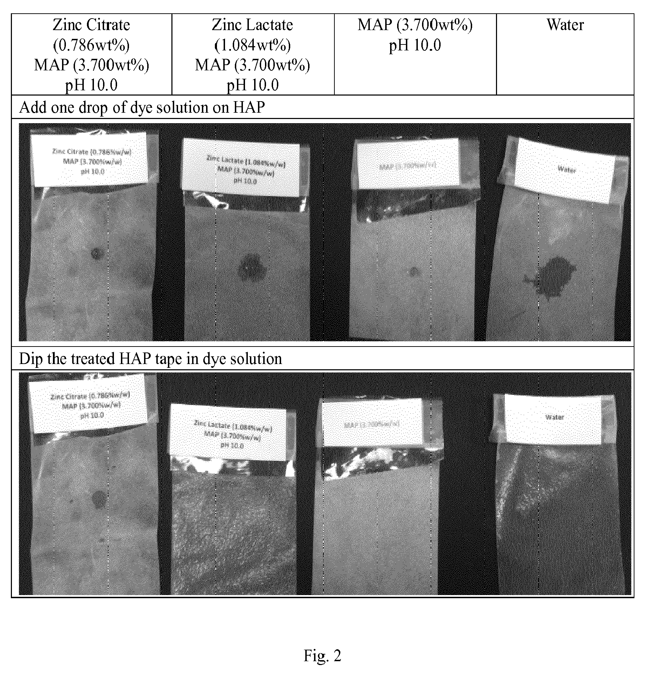

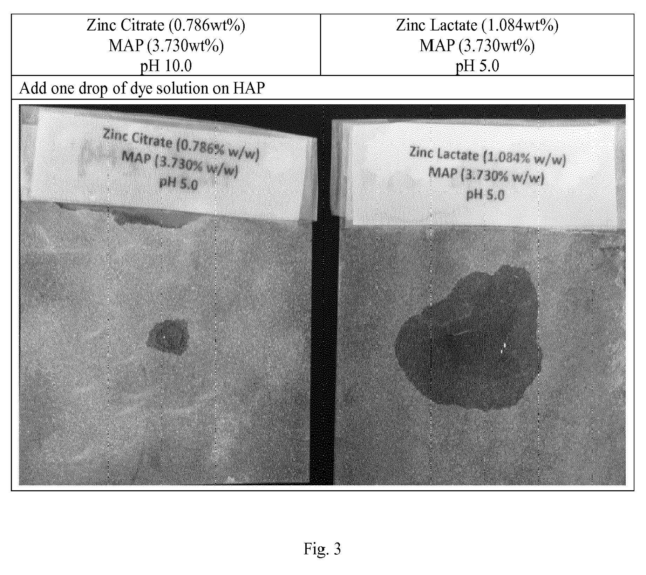

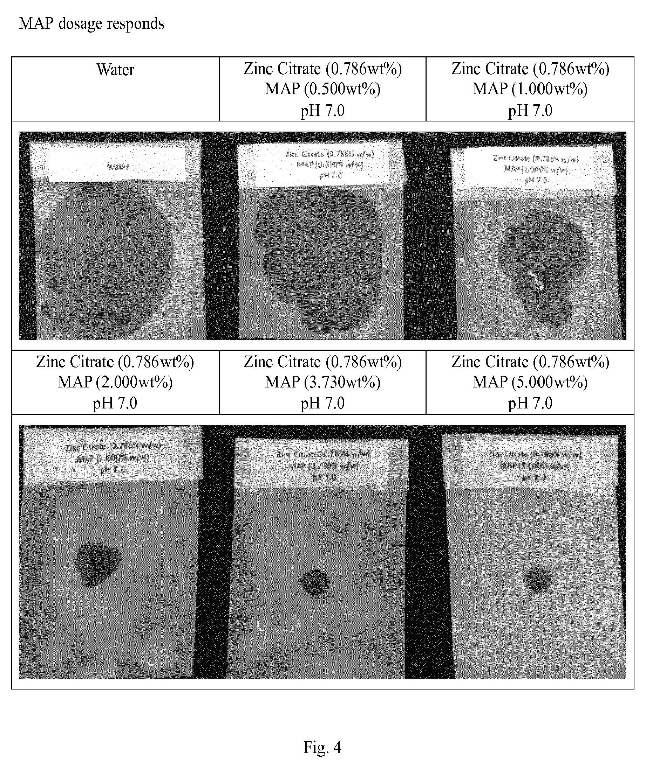

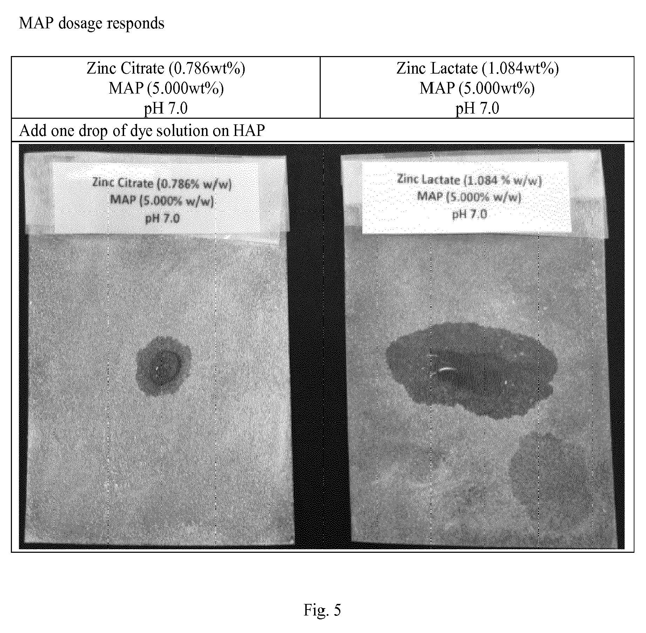

Figs. 1 to 5 demonstrate the stain-proof efficacy of some specific embodiments.

### DETAILED DESCRIPTION OF THE INVENTION

All percentages and ratios used herein are by weight of total composition, unless otherwise indicated. All percentages, ratios, and levels of ingredients referred to herein are based on the actual amount of the ingredient, and do not include solvents, fillers, or other materials with which the ingredient may be combined as a commercially available product, unless otherwise indicated.

All measurements referred to herein are made at room temperature of about 25°C, unless otherwise specified.

The terms "oral composition" and "oral care composition" are used interchangeably herein, and refer to a product, which in the ordinary course of usage, is not intentionally swallowed for purposes of systemic administration of particular therapeutic agents, but is rather retained in the oral cavity for a time sufficient to contact substantially all of the dental surfaces and/or oral tissues for purposes of oral activity. The oral composition may be in various forms including toothpaste, dentifrice, tooth gel, subgingival gel, mouthrinse, mousse, foam, denture product, mouthspray, lozenge, chewable tablet or chewing gum. The oral composition may also be incorporated onto strips or films for direct application or attachment to oral surfaces.

The term "dentifrice", as used herein, includes paste, gel, liquid, powder or tablet formulations unless otherwise specified. The dentifrice composition may be a single phase composition or may be a combination of two or more separate dentifrice compositions. The dentifrice composition may be in any desired form, such as deep striped, surface striped, multilayered, having a gel surrounding a paste, or combinations thereof. Each dentifrice composition in a dentifrice comprising two or more separate dentifrice compositions may be contained in a physically separated compartment of a dispenser and dispensed side-by-side.

The term "teeth", as used herein, refers to natural teeth as well as artificial teeth or dental prosthesis.

The term "orally-acceptable carrier" refer to safe and effective materials and conventional additives used in oral care compositions collectively referred to as "oral care agents" and including but not limited to one or more of fluoride ion sources, anticalculus or anti-tartar agents, antimicrobial agents, anti dry mouth agents, buffers, abrasives such as silica, alkali metal bicarbonate salts, thickening materials, humectants, water, surfactants, titanium dioxide, flavorants, sweetening agents, coolants and other sensates, xylitol, and coloring agents.

Active and other ingredients useful herein may be categorized or described by their cosmetic and/or therapeutic benefit or their postulated mode of action or function. However, it is to be understood that the active and other ingredients useful herein can, in some instances, provide more than one cosmetic and/or therapeutic benefit or function or operate via more than one mode of action. Therefore, classifications herein are made for the sake of convenience and are not intended to limit an ingredient to the particularly stated application or applications listed.

As used herein, the articles including "a" and "an" when used in a claim, are understood to mean one or more of what is claimed or described.

As used herein, the terms "comprise", "comprises", "comprising", "include", "includes", "including", "contain", "contains", and "containing" are meant to be non-limiting, i.e., other steps and other sections which do not affect the end of result can be added. The above terms encompass the terms "consisting of" and "consisting essentially of".

As used herein, the words "preferred", "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

In accordance with the present invention, compositions are provided that comprise a zinc source selected from the group consisting of zinc citrate, zinc citrate dehydrate, and mixtures thereof and one or more organophosphate compounds to impart hydrophobicity to teeth and other oral surfaces. Suitable organophosphate compounds have a strong affinity particularly for the tooth surface and have sufficient surface binding propensity to desorb pellicle proteins and remain affixed thereon. The phosphate groups of the organophosphate attach themselves to cations, in particular calcium ions in teeth or other positively charged sites such as protein residues on the mucosal surface and thus serve to anchor the hydrophobic portion of the molecule onto the surface, thereby modifying it to be hydrophobic. The phosphate groups readily bond to cationic and charged surfaces via electrostatic interaction, hydrogen bonding, or complexation, which leads to ready deposition of the organophosphate upon application to form a coating on the treated surface. The strong bond results in longer retention or durability and substantivity of the coating. The present invention provides oral care compositions that deposit a substantive hydrophobic coating on teeth or other oral surface that is retained for a sufficient period of time to deliver the desired benefit(s), particularly with repeated use. Advantageously, the present organophosphates can also act as a carrier for other active agents such as for example, antimicrobials and cosmetic ingredients, in particular those which are hydrophobic in nature, such as most flavor and fragrance ingredients, delivering such agents to the surface where they can perform their intended function.

The substantive hydrophobic coating functions as protective barrier to prevent access to tooth surfaces by bacteria, acids, food particles, staining agents, etc., and to prevent active agents deposited on the surface from being rapidly washed away. Specifically, the substantive nature of the coating means it is retained longer on the treated surface as opposed to being easily washed away. Substantivity is important because it allows for enduring protection and prolonged contact of the active agents with the surface being treated thereby enhancing the bleaching, antimicrobial, anticaries, taste or cooling sensation or other desired effect on the surface. For example, prolonged retention of antimicrobials on oral surfaces will result in reducing oral microorganisms that are causative of, or associated with, various dental diseases, including gingivitis, periodontal disease, and dental plaque. With respect to fluoride delivery from a daily use oral care composition such as dentifrice or mouthrinse, the present method of using a substantive hydrophobic coating to retain the fluoride that has been deposited thereon represents a means to increase remineralization and fluoride uptake into teeth, which lead to strengthening of the tooth structure and reduction of mineral loss and tooth decay. In another example, delivery and retention of coolants on oral surfaces provide long lasting fresh mouth feel.

In one aspect oral care compositions are provided, which comprise in an orally acceptable carrier from about 0.01% to about 35%, alternatively from about 0.035% to about 20%, alternatively from about 0.035% to about 10%, or alternatively from about 0.035% to about 5%, or 0.125% to 2.5% by weight of the total oral composition of an organophosphate compound, the compositions depositing a substantive hydrophobic coating on oral cavity surfaces, teeth in particular. By depositing a "substantive hydrophobic coating" on a surface is meant that the hydrophobic character of the surface is increased as measured, for example, by an increase in the water contact angle of the surface of at least about 10 degrees and the increased hydrophobic character is maintained for a period of at least about 5 minutes and desirably longer. For example, the water contact angle of dental enamel after treatment with a composition may increase by about 15 degrees or more depending on a number of factors including the chemical nature and solubility characteristics of the organonophosphate, pH, the condition of the oral environment, and tooth surface characteristics.

Examples of suitable organophosphate compounds are mono-, di- or triesters represented by the following general structure wherein Z¹, Z², or Z³ may be identical or different, at least Z¹ being an organic moiety preferably selected from linear or branched alkyl, alkenyl, alkoxylated alkyl or alkoxylated alkenyl group of from 6 to 22 carbon atoms or from 10 to 22 carbons, optionally substituted by one or more phosphate groups; each of Z² and Z³ represents hydrogen, alkali metal, ammonium, protonated alkyl amine, protonated alkanolamine, or a Z¹ group.

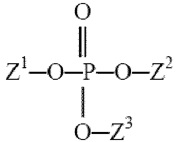

Among suitable organophosphate compounds are alkoxylated alkyl or alkoxylated alkenyl phosphate esters represented by the following structure: 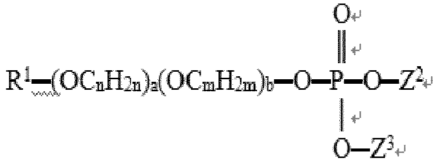 wherein R¹ represents a linear or branched, alkyl or alkenyl group of from 6 to 22 carbon atoms, optionally substituted by one or more phosphate groups; n and m, are individually and separately, 2 to 4; a and b, individually and separately, are 0 to 20, a+b is at least 1 and; Z² and Z³ may be identical or different, each represents hydrogen, alkali metal, ammonium, protonated alkyl amine or protonated functional alkyl amine such as an alkanolamine, or a R¹-(OCnH₂ₙ)ₐ(OCₘH₂ₘ)_{b}- group. In some embodiments, R¹ will desirably be an alkyl group of from 10 to 22 carbon atoms and a and b may each be no more than 10 (a+b ≤ 10) in order to maintain overall hydrophobic character of the organophosphate and the degree of hydrophobicity imparted to the surface.

Examples of organophosphate compounds include mono- di- and tri- alkyl or alkyl (poly)alkoxy phosphates such as dodecyl phosphate, lauryl phosphate; laureth-1 phosphate; laureth-3 phosphate; laureth-9 phosphate; dilaureth-10 phosphate; trilaureth-4 phosphate; C12-18 PEG-9 phosphate and salts thereof. Many are commercially available from suppliers including Croda; Rhodia; Nikkol Chemical; Sunjin; Alzo; Huntsman Chemical; Clariant and Cognis.

Particularly useful organophosphates herein are those that are compatible and stable with other components of oral care composition such as fluoride sources and antimicrobials such as cetylpyridinium chloride (CPC), domiphen bromide and metal ions such as stannous, copper and zinc, thus permitting simple single phase formulations. Even more importantly, the organophosphate agent will not significantly interfere with the activity of other actives in the composition, specifically their fluoridation, mineralization and antimicrobial activities.

In some embodiments, the organophosphate will be used in combination with one or more hydrophobic materials to further increase hydrophobicity and/or improve functionality of the coating deposited on the treated surface. The hydrophobic coating on the surface created by the organophosphate enables deposition of other hydrophobic material(s) resulting in increased hydrophobicity and/or modification of the coating such as for example in terms of continuity and thickness. A continuous hydrophobic coating would provide a more effective protective barrier as well as effectively fill in irregularities, cracks, and crevices on the tooth surface.

Hydrophobic materials useful herein include compounds that are generally non-polar and water-insoluble but may be water dispersible. By "water-insoluble" herein is meant the compound has a solubility in water of about 0.01% or less at 25°C. Suitable hydrophobic or water-insoluble materials include long chain hydrocarbon waxes and oils such as petrolatum and microcrystalline wax; fatty compounds including alcohols, ethers, acids and esters; silicone polymers; and fluoroorganopolymers. Such materials have been suggested for inclusion in oral hygiene preparations.

For example, oral compositions containing silicone oils such as polydimethylsiloxanes (PDMS) are described in U.S. Patent Nos. 5,032,387; 5,165,913; 5,057,308 all to Hill, et al. and in U.S. Patent No. 5,422,098 to Rolla et al. However, PDMS polymers have not generally been used successfully for coating the teeth because of poor adhesion and retention of the PDMS on tooth surfaces. To improve the adherence of the silicone on surfaces, it has been suggested to modify the silicone by addition of functional groups such as carboxy, anhydride, polyol and amino groups. Such modified silicones have been suggested for modifying various surfaces; including fibers, textiles, leather, hair and skin, teeth, paper, plastic, wood, metal, glass, stone and concrete. For example, aminoalkyl silicones are described in commonly assigned US Patent Nos. 6,153,567; 6,129,906 and 6,024,891; carboxy or anhydride group containing silicones are described in commonly assigned US Patent Nos. 7,025,950 and 7,166,235.

Hydrophobic or water-insoluble materials for use herein include long chain hydrocarbons, especially paraffins having a chain length of 16 carbons or greater; natural waxes of animal, vegetable or mineral origin such as beeswax, lanolin, spermaceti, and carnauba wax; and synthetic ethylenic polymers such as polymethylene wax (Paraflint), polybutene and polyisobutene. Also useful are various fluoroorganopolymers where some or all of the hydrogen are replaced by fluorine, including, among others: polytetrafluoroethylene (PTFE); fluorinated polyethylene-propylene (FEP); polyvinylidene fluoride (PVDF); and polyvinylfluoride (PVF). These hydrophobic materials and their use in oral care compositions are described for example in US Patent Nos. 5,665,333; 5,888,480 5,961,958; and 5,980,868 all to Homola et al. Oral care compositions containing polybutene are disclosed e.g., in US Patent Nos. 6,514,484 and 6,719,995 to Rajaiah et al.

Suitable fatty compounds such as described in commonly assigned application published as US 2008/0081023, include those having a hydrophobic tail group R₁, which is an alkyl, alkenyl (containing up to 3 double bonds), alkyl aromatic, or branched alkyl group typically of C₁₂-C₇₀ length. Non-limiting examples of alkyl, alkenyl, or branched alkyl groups suitable for the fatty compounds of the present invention include lauryl, tridecyl, myristyl, pentadecyl, cetyl, heptadecyl, stearyl, arachidyl, behenyl, undecylenyl, palmitoleyl, oleyl, palmoleyl, linoleyl, linolenyl, arahchidonyl, elaidyl, elaeostearyl, erucyl, isolauryl, isotridecyl, isomyristal, isopentadecyl, petroselinyl, isocetyl, isoheptadecyl, isostearyl, isoarachidyl, isobehnyl, gadoleyl, brassidyl, and technical-grade mixture thereof. The alkyl, alkenyl or branched carbon chains may be of vegetable origin.

Suitable fatty compounds of the present invention may also have a hydrophilic head group which does not make the compound water soluble, such as in compounds having a hydrophilic lipophilic balance (HLB) of 6 or less. Non-limiting examples of classes of compounds having such a hydrophilic head group include fatty alcohols, alkoxylated fatty alcohols, fatty phenols, alkoxylated fatty phenols, fatty amides, alkyoxylated fatty amides, fatty amines, fatty alkylamidoalkylamines, fatty alkyoxyalted amines, fatty carbamates, fatty amine oxides, fatty acids, alkoxylated fatty acids, fatty diesters, fatty sorbitan esters, fatty sugar esters, methyl glucoside esters, fatty glycol esters, mono, di and tri glycerides, polyglycerine fatty esters, alkyl glyceryl ethers, propylene glycol fatty acid esters, cholesterol, ceramides, fatty silicone waxes, fatty glucose amides, and phospholipids.

The following provides non-limiting examples of classes of compounds from which one or more fatty compounds suitable for use in the present invention may be selected.

### a. Fatty Alcohols / Alkoxylated Fatty Alcohol Ethers according to the following formula:

**R₁-(OR₂)ₖ-OH**

wherein R₁ is as described above; R₂ is a C₁-C₅ carbon chain which maybe branched or hydroxy substituted; and k is a number ranging from about 0 to about 5.

The fatty alcohols useful herein typically have from about 12 to about 60 carbon atoms, alternatively from about 16 to about 60 carbon atoms. These fatty alcohols may be straight or branched chain alcohols and may be saturated or unsaturated. Non-limiting examples of suitable fatty alcohols include cetyl alcohol, stearyl alcohol, arachidyl alcohol, behenyl alcohol, eicosyl alcohol, C20-40 alcohols, C30-50 alcohols, C40-60 alcohols, and mixtures thereof.

Suitable alkoxylated fatty alcohol ethers include addition products of 1 to 5 mol of ethylene oxide with a linear fatty alcohol having about 12 to about 60 carbon atoms, which are all adducts obtainable by the known industrial oxyethylation processes. Also suitable are the polyethylene oxide condensates of alkyl phenols, for example, the condensation products of alkyl phenols having an alkyl group containing from about 12 to about 60 carbon atoms in either a straight chain or branched chain configuration, with ethylene oxide, wherein the ethylene oxide is present in amounts equal to from about 1 to about 5 moles of ethylene oxide per mole of alkyl phenol. Further suitable alkoxylated fatty alcohol ethers include those derived from the condensation of ethylene oxide with the product resulting from the reaction of propylene oxide and ethylene diamine products.

Non-limiting examples of suitable alkoxylated fatty alcohol ethers include steareth-2, beheneth-2, beheneth-5, beheneth-10, C20-40 Pareth-3, C20-40 Pareth-10, C30-50 Pareth-3, and C30-50-Pareth-10.

### b. Di-Fatty Ethers according to the following formula:

**R₁-(OR₂)ₖ-Z-(R₂O)-R₁**

wherein R₁ is as described above; R₂ is a C₁-C₅ carbon chain which can be branched or hydroxy substituted; k and 1 each is independently a number such that the sum (k + 1) has a value ranging from 1 to 30; and Z is an ether (i.e., -O-) or an amine (i.e.,-NR₂-, wherein R₂ is as described immediately above).

Non-limiting examples of suitable di-fatty ether compounds include dicetylstearyl ether, dicetylstearyl dioxyethyl ether, and N,N-bis(2-cetylstearyloxyethyl)aminoethanol.

### c. Fatty Amides / Fatty Alkanolamides / Fatty Alkoxylated Amides according to the following formula:

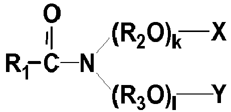

wherein R₁ is as described above; R₂ and R₃ each is independently a C₁-C₅ carbon chain which can be branched or hydroxy substituted; k and 1 each is independently a number such that the sum (k + 1) has a value ranging from 0 to 10; and X and Y are each independently selected from hydrogen, a C₁-C₄ carbon chain which can be branched or hydroxy substituted, morpholine, or a C₅-C₅₀ carbon chain bonded via an amide, ester, or ether linkage.

Non-limiting examples of suitable fatty amides, fatty alkanolamides or fatty alkoxylated amides include Cocamide, Cocamide Methyl MEA, Cocoyl Glutamic Acid, Erucamide, Lauramide, Oleamide, Palmitamide, Stearamide, Stearyl Erucamide, Behenamide DEA, Behenamide MEA, Cocamide DEA, Cocamide MEA, Cocamide MIPA, Hydroxyethyl Stearamide-MIPA, Hydroxypropyl Bisisostearamide MEA, Hydroxypropyl Bislauramide MEA, Hydroxystearamide MEA, Isostearamide DEA, Isostearamide MEA, Isostearamide MIPA, Lauramide DEA, Lauramide MEA, Lauramide MIPA, Myristamide DEA, Myristamide MEA, Myristamide MIPA, Palmamide DEA, Palmamide MEA, Palmamide MIPA, Palmitamide DEA, Palmitamide MEA, PEG-20 Cocamide MEA, Stearamide AMP, Stearamide DEA, Stearamide DEA-Distearate, Stearamide DIBA-Stearate, Stearamide MEA, Stearamide MEA-Stearate, Stearamide MIPA, PEG-2 Cocamide, PEG-3 Cocamide, PEG-4 Cocamide, PEG-5 Cocamide, PEG-6 Cocamide, PEG-7 Cocamide, PEG-3 Lauramide, PEG-5 Lauramide, PEG-3 Oleamide, PEG-9 Oleamide, PEG-4 Stearamide, PEG-10 Stearamide, PPG-2 Cocamide, PPG-2 Hydroxyethyl Cocamide, PPG-2 Hydroxyethyl Coco/Isostearamide, Ceramide 1, Ceramide 2, Ceramide 3, Ceramide 4, and Ceramide 5.

### d. Fatty Carbamates according to the following formula:

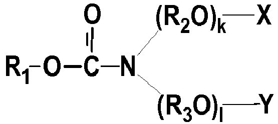

wherein R₁ is as described above; R₂ and R₃ each is independently a C₁-C₅ carbon chain which can be branched or hydroxy substituted; k and 1 each is independently a number such that the sum (k + 1) has a value ranging from 0 to 10; and X and Y each is independently selected from hydrogen, a C₁-C₄ carbon chain which can be branched or hydroxy substituted, morpholine, or a C₅-C₅₀ carbon chain bonded via an amide, ester, or ether linkage.

Non-limiting examples of suitable fatty carbamates include cetyl carbamate, stearyl carbamate, PEG-2 stearyl carbamate, PEG-4 stearyl carbmate, and behenyl carbamate.

### e. Fatty Alkylamido Alkylamines according to the following formula:

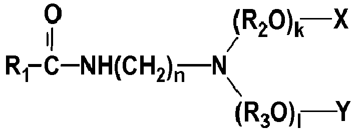

wherein R₁ is as described above; R₂ and R₃ each is independently a C₁-C₅ carbon chain which can be branched or hydroxy substituted; k and 1 each is independently a number such that the sum (k + 1) has a value ranging from 0 to 10; X and Y each is independently selected from hydrogen, a C₁-C₄ carbon chain which can be branched or hydroxy substituted, morpholine, or a C₅-C₅₀ carbon chain bonded via an amide, ester, or ether linkage; and n is a number ranging from about 1 to about 4.

Non-limiting examples of suitable fatty alkylamido alkylamine compounds include stearamidoethyl diethanolamine, stearamidopropyl morpholine, stearamidopropyl dimethylamine stearate, stearamidopropyl dimethylamine, stearamidoethyl diethylamine, stearamidoethyl diethanolamine, isostearamidomorpholine stearate behenamidopropyldimethylamine, behenamidopropyldiethylamine, behenamidoethyldiethyl-amine, cocamidopropyl dimethylamine behenamidoethyldimethylamine, arachidamidopropyldimethylamine, arachidamido-propyidiethylamine, arachidamidoethyidiethylamine, arachidamidoethyidimethylamine, and mixtures thereof.

### f. Fatty Amines / Fatty Alkanolamines / Fatty Alkoxylated Amines according to the following formulas:

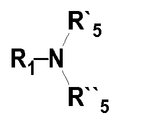

wherein R₁ is as described above; and R'₅ and R"₅ are independently hydrogen or a C₁-C₅ carbon chain which can be branched or hydroxy substituted, 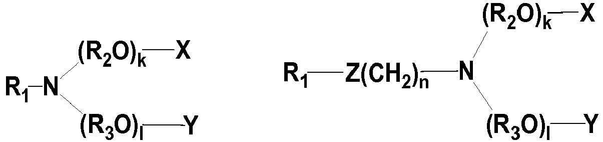 wherein R₁ is as described above; R₂ and R₃ each is independently a C₁-C₅ carbon chain which can be branched or hydroxy substituted; k and 1 each is independently a number such that the sum (k + 1) has a value ranging from 0 to 10; X and Y each is independently hydrogen, a C₁-C₄ carbon chain which can be branched or hydroxy substituted, morpholine, or a C₅-C₅₀ carbon chain bonded via amide, ester, or ether linkage; n is a number ranging from about 1 to about 4; and Z is an ether (i.e., -O-) or an amine (i.e., -NH-).

Primary, secondary, and tertiary fatty amines are useful. Suitable fatty alkoxylated amine compounds include addition products of ethylene oxide with a linear fatty alkylamine having 12 to 60 carbon atoms, all of which are adducts obtainable by known industrial processes and which are commercially available.

Non-limiting examples of suitable fatty amine and fatty alkoxylated amine compounds include diethyllauramine, dicocamine, dimethylcocamine amine cetamine, stearamine, oleamine, behenamine, dimethylbehenamine amine, diethylbehenamine, dibehenylamine N-lauryl diethanolamine. TEA-diricinoleate, TEA-lauryl ether, diethylaminoethyl PEG-5 cocoate, diethylaminoethyl PEG-5 laurate, hydroxyethyl isostearyloxy isopropanolamine, PEG-2 cocamine, PEG-5 cocamine, PEG-10 cocamine, PEG-5 isodecyloxypropylamine, PEG-2 lauramine, PEG-2 oleamine, PEG-5 oleamine, PEG-10 oleamine, PEG-2 stearamine, PEG-5 stearamine, PEG-10 stearamine, PPG-2 cocamine, PPG-2 hydrogenated tallowamine, PPG-2 tallowamine, and PPG-3 tallow aminopropylamine.

### g. Fatty Amine Oxides according to the following formula:

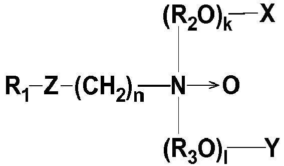

wherein R₁ is as described above; R₂ and R₃ each is independently a C₁-C₅ carbon chain which can be branched or hydroxy substituted; k and 1 each is independently a number such that the sum (k + 1) has a value ranging from 0 to 10; X and Y each is independently hydrogen, a C₁-C₄ carbon chain which can be branched or hydroxy substituted, morpholine, or a C₅-C₅₀ carbon chain bonded via an amide, ester, or ether linkage; Z is an ether (i.e., -O-) or an amide (i.e., -C(O)-NH-) linkage; and n is a number ranging from about 1 to about 4. In accord with known convention, the arrow in the above formula is representative of a semi-polar bond.

Non-limiting examples of suitable amine oxide compounds include dimethyldodecylamine oxide, oleyldi(2-hydroxyethyl) amine oxide, dimethyltetradecylamine oxide, di(2-hydroxyethyl)-tetradecylamine oxide, dimethylhexadecylamine oxide, behenamine oxide, cocamine oxide, decyltetradecylamine oxide, dihydroxyethyl C 12-15 alkoxypropylamine oxide, dihydroxyethyl cocamine oxide, dihydroxyethyl lauramine oxide, dihydroxyethyl stearamine oxide, dihydroxyethyl tallowamine oxide, hydrogenated palm kernel amine oxide, hydrogenated tallowamine oxide, hydroxyethyl hydroxypropyl C12-15 alkoxypropylamine oxide, lauramine oxide, myristamine oxide, myristyl/cetyl amine oxide, oleamidopropylamine oxide, oleamine oxide, palmitamine oxide, PEG-3 lauramine oxide, potassium trisphosphonomethylamine oxide, stearamine oxide, and tallowamine oxide.

### h. Fatty Acid / Alkoxylated Fatty Acid according to the following formula:

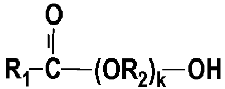

wherein R₁ is as described above; R₂ is a C₁-C₅ carbon chain which can be branched or hydroxy substituted; and k is a number ranging from about 0 to about 5.

Non-limiting examples of suitable fatty acids and alkoxylated fatty acids include behenic acid, C10-40 hydroxyalkyl acid, C32-36 isoalkyl acid coconut acid, erucic acid, hydroxystearic acid, lauric acid, linoleic acid, myristic acid, oleic acid, palmitic acid, PEG-8 behenate, PEG-5 cocoate, PEG-10 cocoate, PEG-2 laurate, PEG-4 laurate PEG-6 laurate, PEG-8 laurate, PEG-9 laurate, PEG-10 laurate, PEG-7 oleate, PEG-2 stearate, PEG-3 stearate, PEG-4 stearate, PEG-5 stearate, PEG-6 stearate, PEG-7 stearate, PEG-8 stearate, PEG-9 stearate, PEG-10 stearate, polyglyceryl-2-PEG-4 stearate, PPG-2 isostearate, and PPG-9 laurate.

### i. Fatty Esters according to the following formula:

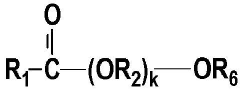

wherein R₁ is as described above; R₂ is a C₁-C₅ carbon chain which can be branched or hydroxy substituted; k is a number ranging from about 1 to about 5; and R₆ is a C₁-C₄₀ carbon chain or an alkylcarbonyl (i.e., -C(O)-R₇, wherein R₇ is a C₁-C₄₀ carbon chain).

These suitable fatty esters include esters with hydrocarbyl chains derived from fatty acids or alcohols (e.g., mono-esters, polyhydric alcohol esters, and di- and tricarboxylic acid esters). The hydrocarbyl radicals of the fatty esters hereof may include or have covalently bonded thereto other compatible functionalities, such as amides and alkoxy moieties (e.g., ethoxy or ether linkages, etc.).

Non-limiting examples of suitable fatty ester compounds include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Fatty ester compounds of the present invention also may be selected from those according to the following formula: 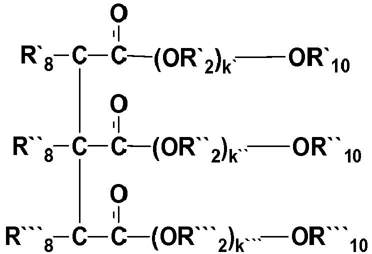 wherein R'₈, R"₈, and R"₈ each is independently selected from hydrogen, hydroxy, or a C₁-C₄ carbon chain which can be branched or hydroxy substituted; k', k", and k'" each is independently a number such that the sum (k'+ k"+ k"') has a value ranging from 0 to 15; R'₂, R"₂, and R"'₂ each is independently selected from a C₁-C₅ carbon chain which can be branched or hydroxy substituted; and where R'₁₀, R"₁₀, R"'₁₀ each is independently selected from hydrogen or R₁, where R₁ is as defined above, provided that at least one of R'₁₀, R"₁₀, and R"'₁₀ is a R₁ group.

Still other suitable fatty esters are di- and tri-alkyl and alkenyl esters of carboxylic acids, such as esters of C₄ to C₈ dicarboxylic acids (e.g., C₁ to C₂₂ esters or C₁ to C₆ esters of succinic acid, glutaric acid, and adipic acid). Specific non-limiting examples of di- and tri- alkyl and alkenyl esters of carboxylic acids include isocetyl stearyol stearate, stearyl citrate, distearyl citrate and tristearyl citrate.

Other useful fatty ester compounds are represented by the following formula: 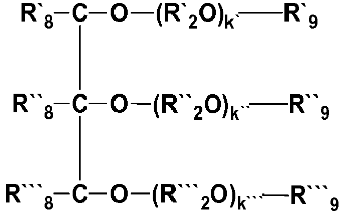 wherein R'₂, R"₂, and R"'₂ each is independently selected from a C₁-C₅ carbon chain which can be branched or hydroxy substituted; R'₈, R"₈, and R"'₈ each is independently selected from hydrogen, hydroxy, or C₁-C₄ carbon chain which can be branched or hydroxy substituted; k', k", and k'" each is independently a number such that the sum (k'+ k"+ k"') has a value ranging from 0 to 15; and R'₉, R"₉, and R"'₉ each is independently selected from hydrogen or alkylcarbonyl (i.e., -C(O)-R₁, wherein R₁ is as described above), provided that at least one of R'₉, R"₉, and R"'₉ is a -C(O)-R₁ group.

Other suitable fatty esters are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester.

Still other fatty esters suitable for use in the compositions of the present invention are glycerides, including, but not limited to, mono-, di-, and tri-glycerides. For use in the compositions described herein, examples of glycerides are the mono-, di-, and tri-esters of glycerol and long chain carboxylic acids, such as C₁₂ to C₂₂ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

### j. Fatty Phosphorus Compounds according to the following formula:

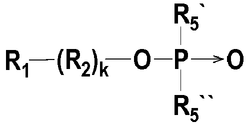

wherein R₁ is as described above; R₂ is a C₁-C₅ carbon chain which can be branched or hydroxy substituted; k is a number ranging from about 0 to about 5; and R₅ is hydrogen or a C₁-C₄ carbon chain which can be branched or hydroxy substituted. In accord with known convention, the arrow in the above formula is representative of a semi-polar bond.

Non-limiting examples of suitable fatty phosphorus compounds include dodecyldimethylphosphine oxide, tetradecyldimethylphosphine oxide, tetradecylmethylethylphosphine oxide, 3,6,9,-trioxaoctadecyldimethylphosphine oxide, cetyldimethylphosphine oxide, 3- dodecoxy-2-hydroxypropyldi(2-hydroxyethyl) phosphine oxide, stearyldimethylphosphine oxide, cetylethylpropylphosphine oxide, oleyldiethylphosphine oxide, dodecyldiethylphosphine oxide, tetradecyldiethylphosphine oxide, dodecyldipropylphosphine oxide, dodecyldi(hydroxymethyl)phosphine oxide, dodecyldi(2-hydroxyethyl) phosphine oxide, tetradecylmethyl-2-hydroxypropylphosphine oxide, oleyldimethylphosphine oxide, and 2-hydroxydodecyldimethylphosphine oxide.

### k. Fatty Sorbitan Derivatives according to the following formula:

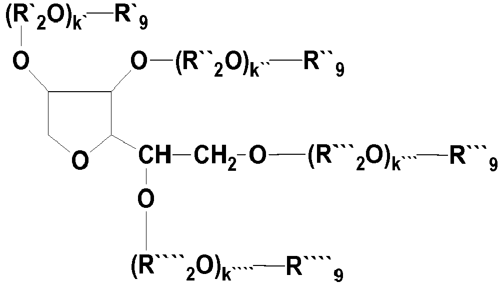

wherein R'₂, R"₂, R"'₂, and R""₂ each is independently a C₁-C₅ carbon chain which can be branched or hydroxy substituted; R'₉, R"₉, R"'₉, and R""₉ each is independently hydrogen or alkylcarbonyl (i.e., -C(O)-R₁, wherein R₁ is as described above), provided that at least one of R'₉, R"₉, R"'₉, and R""₉ is a -C(O)-R₁, group; and k', k", k"', and k"" each is independently a number such that the sum (k' + k" + k'" + k"") has a value of from 0 to 20.

Non-limiting examples of suitable fatty sorbitan derivatives include PEG-20 sorbitan cocoate, PEG-2 sorbitan isostearate, PEG-5 sorbitan isostearate, PEG-20 sorbitan isostearate, PEG-10 sorbitan laurate, PEG-3 sorbitan oleate, PEG-6 sorbitan oleate, PEG-20 sorbitan oleate, PEG-3 sorbitan stearate, PEG-4 sorbitan stearate, PEG-6 sorbitan stearate, PEG-4 sorbitan triisostearate, PEG-20 sorbitan triisostearate, PEG-2 sorbitan trioleate, PEG-3 sorbitan tristearate, polyglyceryl-2 sorbitan tetraethylhexanoate, sorbitan caprylate, sorbitan cocoate, sorbitan diisostearate, sorbitan dioleate, sorbitan distearate, sorbitan isostearate, sorbitan laurate, sorbitan oleate, sorbitan olivate, sorbitan palmitate, sorbitan sesquiisostearate, sorbitan sesquioleate, sorbitan sesquistearate, sorbitan stearate, sorbitan triisostearate, sorbitan trioleate, sorbitan tristearate, and sorbitan undecylenate.

### 1. Sucrose Polyesters according to the following formula:

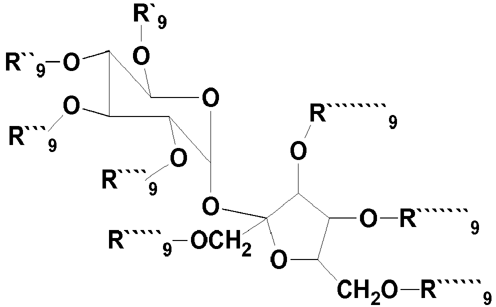

wherein R'₉, R"₉, R"'₉, R""₉, R""'₉, R"""₉, R"""'₉, and R""""₉ each is hydrogen or alkylcarbonyl (i.e., -C(O)-R₁, wherein R₁ is as described above), provided that at least one of R'₉, R"₉, R"'₉, R""₉, R""'₉, R"""₉, R"""'₉, and R""""₉ is a -C(O)-R₁ group.

Non-limiting examples of suitable sucrose polyester compounds include Sucrose Cocoate, Sucrose Dilaurate, Sucrose Distearate, Sucrose Hexaerucate, Sucrose Hexaoleate/Hexapalmitate/Hexastearate, Sucrose Hexapalmitate, Sucrose Laurate, Sucrose Mortierellate, Sucrose Myristate, Sucrose Octaacetate, Sucrose Oleate, Sucrose Palmitate, Sucrose Pentaerucate, Sucrose Polybehenate, Sucrose Polycottonseedate, Sucrose Polylaurate, Sucrose Polylinoleate, Sucrose Polyoleate, Sucrose Polypalmate, Sucrose Polysoyate, Sucrose Polystearate, Sucrose Ricinoleate, Sucrose Stearate, Sucrose Tetraisostearate, Sucrose Tetrastearate Triacetate, Sucrose Tribehenate, and Sucrose Tristearate.

### m. Alkyl Sulfoxides according to the following formula:

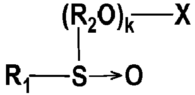

wherein R₁ is as described above; R₂ is a C₁-C₅ carbon chain which can be branched or hydroxy substituted; k is a number ranging from about 0 to about 10; and X and Y each is independently selected from hydrogen or a C₁-C₄ carbon chain which can be branched or hydroxy substituted.

Non-limiting examples of suitable alkyl sulfoxide compounds include octadecyl methyl sulfoxide, 2-ketotridecyl methyl sulfoxide, 3,6,9-trioxaoctadecyl 2-hydroxyethyl sulfoxide, dodecyl methyl sulfoxide, oleyl 3-hydroxypropyl sulfoxide, tetradecyl methyl sulfoxide, 3-methoxytridecyl methyl sulfoxide, 3- hydroxytridecyl methyl sulfoxide, and 3-hydroxy-4-dodecoxybutyl methyl sulfoxide.

The hydrophobic material added in combination with the organophosphate may be incorporated in the present dentifrice, rinse, denture cleanser, chewing gum and the like compositions at about 0.5% to about 20% by weight or from about 0.5% to about 5% by weight. Greater amounts up to about 90% may be used for oral gels such as paint-on or leave-on finishing or sealing gels or for denture adhesives.

### Evaluation of Activity of Compositions

The presence of certain co-surfactants, e.g., sodium lauryl sulfate (SLS); cocamidopropyl betaine (CAPB); and Tergitol™15-S-9, may also affect the degree of hydrophobicity imparted to the surface. The hydrophobicity may be reduced; however this may be advantageous in certain applications, for instance in allowing better penetration of water soluble actives such as fluoride onto the tooth surface while still providing sufficient surface protection.

An increase in the concentration of the organophosphate will generally provide increased hydrophobicity of the tooth surface with a corresponding increase in surface protection against acid attack.

### Orally Acceptable Carriers

The compositions may comprise optional components (collectively referred to as orally acceptable carriers or excipients) which are described in the following paragraphs along with non-limiting examples. These orally acceptable carrier materials include one or more compatible solid or liquid excipients or diluents which are suitable for topical oral administration. By "compatible" is meant that the components of the composition are capable of being commingled without interaction in a manner which would substantially reduce composition stability and/or efficacy. Suitable carriers or excipients are well known in the art. Their selection will depend on secondary considerations like taste, cost, and shelf stability, etc.

### Fluoride Source

A fluoride ion source is typically present in dentifrices and other oral compositions in amounts sufficient to give a fluoride ion concentration in the composition to provide anticaries effectiveness. The fluoride ion source will typically comprise from about 0.0025% to about 5.0% or from about 0.005% to about 2.0% by weight of the composition. As discussed above, prevention of caries is essential for overall tooth health and integrity. A wide variety of fluoride ion-yielding materials can be employed as sources of soluble fluoride. Representative fluoride ion sources include: stannous fluoride, sodium fluoride, potassium fluoride, amine fluoride, sodium monofluorophosphate, indium fluoride and many others.

In commonly assigned application published as US 2008/0247973A1, it is reported that alkyl phosphates can influence fluoride uptake. Alkyl phosphates which make surfaces relatively more hydrophobic provide good surface protection but relatively less fluoride uptake than alkyl phosphates that make surfaces less hydrophobic, such as alkyl phosphates that either have ethoxy groups or large polar counter ions that make them relatively more hydrophilic. Generally, the relatively more hydrophilic organophosphates allow good fluoride uptake and provide acceptable surface protection benefits. Thus formulating compositions comprising organophosphates and fluoride may require careful selection of organophosphate species and/or adjusting the formulation in order to ensure that both anti-caries efficacy from fluoride and surface protection benefits from the organophosphate are delivered.

In one embodiment, a dentifrice product within a single container comprises two thermodynamically stable but separate phases, the first phase comprising a fluoride ion source in an aqueous carrier and the second phase comprising an organophosphate compound in a non-aqueous carrier, wherein the fluoride phase is delivered before the organophosphate phase such that teeth become exposed to fluoride prior to exposure to the organophosphate. The fluoride phase being an aqueous phase would solubilize in the mouth faster than a non-aqueous organophosphate phase.

In another embodiment, a dentifrice product comprises at least two separate phases contained in separate compartments of a dispenser. One compartment contains a fluoride phase and another compartment contains an organophosphate phase. The dual-phase composition provides a means to allow fluoride deposition on the teeth prior to deposition of the organophosphate. In a single phase embodiment containing fluoride and organophosphate together, delayed release of the organophosphate can be accomplished for example, by encapsulating the organophosphate and triggering release of the organophosphate by a pH change, mechanical shear, dilution or other mechanism.

### Antimicrobial Agent

The present compositions may include an antimicrobial agent, such as a quaternary ammonium antimicrobial agent to provide bactericidal efficacy, i.e., effectiveness in killing, and/or altering metabolism, and/or suppressing the growth of, microorganisms which cause topically-treatable infections and diseases of the oral cavity, such as plaque, caries, gingivitis, and periodontal disease.

The antimicrobial quaternary ammonium compounds used in the compositions of the present invention include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, e.g., methyl or ethyl groups. Dodecyl trimethyl ammonium bromide, domiphen bromide, cetylpyridinium chloride (CPC), tetradecylpyridinium chloride, N-tetradecyl-4-ethyl pyridinium chloride, dodecyl dimethyl (2-phenoxyethyl) ammonium bromide, benzyl dimethoylstearyl ammonium chloride, quaternized 5-amino-1,3-bis(2-ethyl-hexyl)-5-methyl hexahydropyrimidine, benzalkonium chloride, benzethonium chloride, methyl benzethonium chloride and bis[4-(R-amino)-1-pyridinium] alkanes are exemplary of typical quaternary ammonium antimicrobial agents. Commonly used pyridinium compounds include cetylpyridinium, or tetradecylpyridinium halide salts (i.e., chloride, bromide, fluoride and iodide). The quaternary ammonium antimicrobial agents may be included in the present compositions at levels of at least about 0.035%, typically from about 0.045% to about 1.0% or from about 0.05% to about 0.10% by weight.

As described in commonly assigned application WO 05/072693, the bioavailability and activity of quaternary ammonium antimicrobials are negatively affected particularly by anionic surfactants, which are common ingredients in oral care formulations. Thus, it is particularly surprising that some of the present surface-active and anionic organophosphate compounds would be compatible with quaternary ammonium antimicrobials such as CPC, in that the bioavailability and antimicrobial activity are not significantly affected.

The present compositions may comprise a metal ion source that provides stannous ions, zinc ions, copper ions, or mixtures thereof as antimicrobial agent. The metal ion source can be a soluble or a sparingly soluble compound of stannous, zinc, or copper with inorganic or organic counter ions. Examples include the fluoride, chloride, chlorofluoride, acetate, hexafluorozirconate, sulfate, tartrate, gluconate, citrate, malate, glycinate, pyrophosphate, metaphosphate, oxalate, phosphate, carbonate salts and oxides of stannous, zinc, and copper.

Stannous, zinc and copper ions have been found effective to reduce gingivitis, plaque, and sensitivity and provide improved breath benefits. An effective amount is defined as from about 50 ppm to about 20,000 ppm metal ion of the total composition or from about 500 ppm to about 15,000 ppm. Typically, metal ions are present in an amount from about 3,000 ppm to about 13,000 ppm or from about 5,000 ppm to about 10,000 ppm. This is the total amount of metal ions (stannous, zinc, copper and mixtures thereof) for delivery to the tooth surface.

Stannous salts include stannous fluoride and stannous chloride typically used in dentifrices. Other suitable stannous salts include stannous acetate, stannous tartrate and sodium stannous citrate. Examples of suitable zinc ion sources are zinc oxide, zinc sulfate, zinc chloride, zinc citrate, zinc lactate, zinc gluconate, zinc malate, zinc tartrate, zinc carbonate, zinc phosphate, and mixtures thereof. Zinc citrate and zinc lactate are commonly used. Examples of suitable copper ion sources are listed in U.S. Pat. No. 5,534,243. The combined metal ion source(s) maybe present in an amount of from about 0.05% to about 11%, by weight of the final composition, from about 0.5 to about 7%, or from about 1% to about 5%. The stannous salts may be present in an amount of from about 0.1 to about 7%, from about 1% to about 5%, or from about 1.5% to about 3% by weight of the total composition. The amount of zinc or copper salts used in the present invention may range from about 0.01 to about 5%, from about 0.05 to about 4%, or from about 0.1 to about 3.0%.

In a specific embodiment, the zinc source can be selected from the group consisting of zinc citrate and any of its hydrated form. In a specific embodiment, the zinc source is zinc citrate dihydrate.

In a specific embodiment, the present oral care composition comprises from 0.01%, 0.03%, 0.05%, 0.06% or 0.08% to 0.2%, 0.5%, 1%, 1.8% or 2.5% by weight of zinc citrate dihydrate. In an alternative embodiment, the present oral care composition comprises from 0.2% or 0.5% to 1% or 1.8% by weight of zinc citrate dihydrate.

In another specific embodiment, the present oral care composition comprises zinc citrate in an amount sufficient to provide from 200 ppm, 500 ppm, 1000 ppm, 1800 ppm, or 2500 ppm to 3000 ppm, 3500 ppm, 4000 ppm, 5000ppm, or 6000 ppm of zinc ion.

Other antimicrobial agents useful herein include non-cationic antimicrobial agents such as halogenated diphenyl ethers, phenolic compounds including phenol and its homologs, mono and poly-alkyl and aromatic halophenols, resorcinol and its derivatives, xylitol, bisphenolic compounds and halogenated salicylanilides, benzoic esters, and halogenated carbanilides. Also useful as antimicrobials are enzymes, including endoglycosidase, papain, dextranase, mutanase, and mixtures thereof. Commonly used antimicrobial agents include chlorhexidine, triclosan, triclosan monophosphate, and essential oils such as thymol. These agents may be present at levels of from about 0.01% to about 1.5%, by weight of the composition.

### Anticalculus Agent

The present compositions may optionally include an anticalculus agent, such pyrophosphate ions provided by pyrophosphate salts such as mono-, di- and tetraalkali metal pyrophosphates. Disodium dihydrogen pyrophosphate (Na₂H₂P₂O₇), sodium acid pyrophosphate, tetrasodium pyrophosphate (Na₄P₂O₇), and tetrapotassium pyrophosphate (K₄P₂O₇) in their unhydrated or hydrated forms are commonly used species. The pyrophosphate salt may be present as predominately dissolved, predominately undissolved, or a mixture of dissolved and undissolved pyrophosphate. In some embodiments the amount of free pyrophosphate ions may be from about 1% to about 15%, from about 1.5% to about 10% or from about 2% to about 6%. Free pyrophosphate ions may be present in a variety of protonated states depending on the pH of the composition.

Compositions comprising predominately undissolved pyrophosphate refer to compositions containing no more than about 20% of the total pyrophosphate salt dissolved in the composition, typically less than about 10% of the total pyrophosphate dissolved in the composition. Tetrasodium pyrophosphate salt is a typical pyrophosphate salt in these compositions, in anhydrous salt form, the decahydrate form, or any other species stable in solid form in the dentifrice compositions. The salt is in its solid particle form, which may be its crystalline and/or amorphous state, with the particle size of the salt preferably being small enough to be aesthetically acceptable and readily soluble during use. The amount of pyrophosphate salt useful in making these compositions is any tartar control effective amount, generally from about 1.5% to about 15%, from about 2% to about 10%, or from about 3% to about 8%, by weight.

Optional agents to be used in place of or in combination with the pyrophosphate salt include such known materials as longer chain polyphosphates (n=3 or more) including tripolyphosphate, tetrapolyphosphate and hexametaphosphate; synthetic anionic polymers, including polyacrylates and copolymers of maleic anhydride or acid and methyl vinyl ether (e.g., Gantrez®), as well as, e.g., polyamino propane sulfonic acid (AMPS), diphosphonates (e.g., EHDP; AHP), polypeptides (such as polyaspartic and polyglutamic acids), and mixtures thereof.

### Other Active Agents

Another active agent that may be included in the present compositions is a tooth bleaching active selected from peroxides, perborates, percarbonates, peroxyacids, persulfates, and combinations thereof. Suitable peroxide compounds include hydrogen peroxide, urea peroxide, calcium peroxide, sodium peroxide, zinc peroxide and mixtures thereof. A commonly used percarbonate is sodium percarbonate A common persulfate is potassium peroxymonosulfate (also known as MPS and the trade names Caroat and Oxone).

Commonly used peroxide sources in dentifrice formulations include calcium peroxide and urea peroxide. Hydrogen peroxide and urea peroxide are typically used in mouthrinse formulations. The present composition may contain from about 0.01% to about 30%, from about 0.1% to about 10%, or from about 0.5% to about 5% of a peroxide source, by weight.

In addition to whitening, the peroxide also provides other benefits to the oral cavity, including curative and/or prophylactic treatment of caries, dental plaque, gingivitis, periodontitis, mouth odor, recurrent aphthous ulcers, denture irritations, orthodontic appliance lesions, post-extraction and post-periodontal surgery, traumatic oral lesions and mucosal infections, herpetic stomatitis and the like.

Another optional active agent that may be added to the present compositions is a dentinal desensitizing agent to control hypersensitivity, such as salts of potassium, calcium, strontium and tin including nitrate, chloride, fluoride, phosphates, pyrophosphate, polyphosphate, citrate, oxalate and sulfate.

The present compositions may optionally include nutrients particularly those that improve the condition of the oral cavity, such as minerals, vitamins, and amino acids. Amino acids useful in the composition of the invention include basic amino acids such as arginine, lysine, histidine, their salts and/or combinations thereof. Examples of minerals include calcium, phosphorus, fluoride, zinc, manganese, potassium and mixtures thereof. In particular calcium salts may be included in the present compositions to provide mineralization and tooth strengthening benefits.

Once deposited on the tooth surface, the organophosphate coating would prevent these active agents from being rapidly washed away.

### Tooth Substantive Agent

The present invention may include a tooth substantive agent such as polymeric surface active agents (PMSA's), which are polyelectrolytes, for example, anionic polymers, provided they do not significantly affect the desired hydrophobic modification herein. The PMSA's contain anionic groups, e.g., phosphate, phosphonate, carboxy, or mixtures thereof, and thus, have the capability to interact with cationic or positively charged entities. The "mineral" descriptor is intended to convey that the surface activity or substantivity of the polymer is toward mineral surfaces such as calcium phosphate minerals in teeth.

PMSA's are useful in the present compositions because of their stain prevention benefit. It is believed the PMSA's provide a stain prevention benefit because of their reactivity or substantivity to mineral surfaces, resulting in desorption of portions of undesirable adsorbed pellicle proteins, in particular those associated with binding color bodies that stain teeth, calculus development and attraction of undesirable microbial species. The retention of these PMSA's on teeth can also prevent stains from accruing due to disruption of binding sites of color bodies on tooth surfaces.

The ability of PMSA's to bind stain promoting ingredients of oral care products, for example, stannous ions and cationic antimicrobials, is also believed to be helpful. The PMSA will also provide tooth surface conditioning effects which produce desirable effects on surface thermodynamic properties and surface film properties, which impart improved clean feel aesthetics both during and most importantly, following rinsing or brushing. Many of these polymeric agents are also known or expected to provide tartar control benefits when applied in oral compositions, hence providing improvement in both the appearance of teeth and their tactile impression to consumers.

Suitable examples of PMSA's are polyelectrolytes such as condensed phosphorylated polymers; polyphosphonates; copolymers of phosphate- or phosphonate-containing monomers or polymers with other monomers such as ethylenically unsaturated monomers and amino acids or with other polymers such as proteins, polypeptides, polysaccharides, poly(acrylate), poly(acrylamide), poly(methacrylate), poly(ethacrylate), poly(hydroxyalkylmethacrylate), poly(vinyl alcohol), poly(maleic anhydride), poly(maleate) poly(amide), poly(ethylene amine), poly(ethylene glycol), poly(propylene glycol), poly(vinyl acetate) and poly(vinyl benzyl chloride); polycarboxylates and carboxy-substituted polymers; and mixtures thereof. Suitable polymeric mineral surface active agents include the carboxy-substituted alcohol polymers described in U.S. Patent Nos. 5,292,501; 5,213,789, 5,093,170; 5,009,882; and 4,939,284; all to Degenhardt et al. and the diphosphonate-derivatized polymers in U.S. Patent 5,011,913 to Benedict et al; the synthetic anionic polymers including polyacrylates and copolymers of maleic anhydride or acid and methyl vinyl ether (e.g., Gantrez®), as described, for example, in U.S. Patent 4,627,977, to Gaffar et al. Diphosphonate modified polyacrylic acid is one example. Polymers with activity must have sufficient surface binding propensity to desorb pellicle proteins and remain affixed to enamel surfaces. For binding to tooth surfaces, polymers with end or side chain phosphate or phosphonate functions are effective although other polymers with mineral binding activity may also be effective depending upon adsorption affinity.

Additional examples of suitable phosphonate containing polymeric mineral surface active agents include the geminal diphosphonate polymers disclosed as anticalculus agents in US 4,877,603 to Degenhardt et al; phosphonate group containing copolymers disclosed in US 4,749,758 to Dursch et al. and in GB 1,290,724 (both assigned to Hoechst) suitable for use in detergent and cleaning compositions; and the copolymers and cotelomers disclosed as useful for applications including scale and corrosion inhibition, coatings, cements and ion-exchange resins in US 5,980,776 to Zakikhani et al. and US 6,071,434 to Davis et al. Additional polymers include the water-soluble copolymers of vinylphosphonic acid and acrylic acid and salts thereof disclosed in GB 1,290,724 wherein the copolymers contain from about 10% to about 90% by weight vinylphosphonic acid and from about 90% to about 10% by weight acrylic acid, more particularly wherein the copolymers have a weight ratio of vinylphosphonic acid to acrylic acid of 70% vinylphosphonic acid to 30% acrylic acid; 50% vinylphosphonic acid to 50% acrylic acid; or 30% vinylphosphonic acid to 70% acrylic acid. Other suitable polymers include the water soluble polymers disclosed by Zakikhani and Davis prepared by copolymerizing diphosphonate or polyphosphonate monomers having one or more unsaturated C=C bonds (e.g., vinylidene-1,1-diphosphonic acid and 2-(hydroxyphosphinyl)ethylidene-1,1-diphosphonic acid), with at least one further compound having unsaturated C=C bonds (e.g., acrylate and methacrylate monomers). Examples of suitable polymers include the diphosphonate/acrylate polymers supplied by Rhodia under the designation ITC 1087 (Average MW 3000-60,000) and Polymer 1154 (Average MW 6000-55,000).

Among useful PMSA's herein are polyphosphates. A polyphosphate is generally understood to consist of two or more phosphate molecules arranged primarily in a linear configuration, although some cyclic derivatives may be present. Although pyrophosphates (n=2) are technically polyphosphates, particularly useful polyphosphates as PMSA are those having around three or more phosphate groups so that surface adsorption at effective concentrations produces sufficient non-bound phosphate functions, which may enhance the anionic surface charge and affect the hydrophilicity of the surfaces. Examples of inorganic polyphosphate salts include tripolyphosphate, tetrapolyphosphate and hexametaphosphate, among others. The linear polyphosphates are represented by the formula: XO(XPO₃)ₙX, wherein X is typically sodium, potassium or ammonium and n averages from about 3 to about 125. Commercially available longer-chain polyhosphates having n averaging from about 6 to about 21 include those known as Sodaphos (n≈6), Hexaphos (n≈13), and Glass H (n≈21) and are supplied by FMC Corporation and Astaris. Polyphosphates are susceptible to hydrolysis in high water formulations at acid pH, particularly below pH 5. Thus longer-chain polyphosphates are particularly useful, such as Glass H. It is believed longer-chain polyphosphates when undergoing hydrolysis produce shorter-chain polyphosphates which are still effective to deposit onto teeth and provide a stain preventive benefit.

Other polyphosphorylated compounds may be used in addition to or instead of the polyphosphate, in particular polyphosphorylated inositol compounds such as phytic acid also known as myo-inositol 1,2,3,4,5,6-hexakis (dihydrogen phosphate) or inositol hexaphosphoric acid; myo-inositol pentakis(dihydrogen phosphate); myo-inositol tetrakis(dihydrogen phosphate), myo-inositol trikis(dihydrogen phosphate), and an alkali metal, alkaline earth metal or ammonium salt thereof. Herein, the term "phytate" includes phytic acid and its salts as well as other polyphosphorylated inositol compounds.

The amount of tooth substantive agent may range from about 0.1% to about 35% by weight of the total oral composition. In dentifrice formulations, the amount is typically from about 2% to about 30%, from about 5% to about 25%, or from about 6% to about 20%. In mouthrinse compositions, the amount of tooth substantive agent may range from about 0.1% to 5% or from about 0.5% to about 3%.

In addition to creating the surface modifying effects, the tooth substantive agent may also function to solubilize insoluble salts. For example, Glass H has been found to solubilize insoluble stannous salts. Thus, in compositions containing stannous fluoride for example, Glass H contributes to decreasing the stain promoting effect of stannous.

### Chelating agents

Another optional agent is a chelating agent, also called sequestrants, such as gluconic acid, tartaric acid, citric acid and pharmaceutically-acceptable salts thereof. Chelating agents are able to complex calcium found in the cell walls of the bacteria. Chelating agents can also disrupt plaque by removing calcium from the calcium bridges which help hold this biomass intact. However, it is not desired to use a chelating agent which has an affinity for calcium that is too high, as this may result in tooth demineralization, which is contrary to the objects and intentions of the present invention. Suitable chelating agents will generally have a calcium binding constant of about 10¹ to 10⁵ to provide improved cleaning with reduced plaque and calculus formation. Chelating agents also have the ability to complex with metallic ions and thus aid in preventing their adverse effects on the stability or appearance of products. Chelation of ions, such as iron or copper, helps retard oxidative deterioration of finished products.

Examples of suitable chelating agents are sodium or potassium gluconate and citrate; citric acid/alkali metal citrate combination; disodium tartrate; dipotassium tartrate; sodium potassium tartrate; sodium hydrogen tartrate; potassium hydrogen tartrate; sodium, potassium or ammonium polyphosphates and mixtures thereof. The chelating agent may be used from about 0.1% to about 2.5%, from about 0.5% to about 2.5% or from about 1.0% to about 2.5%.

Still other chelating agents suitable for use in the present invention are the anionic polymeric polycarboxylates. Such materials are well known in the art, being employed in the form of their free acids or partially or fully neutralized water soluble alkali metal (e.g., potassium and sodium) or ammonium salts. Examples are 1:4 to 4:1 copolymers of maleic anhydride or acid with another polymerizable ethylenically unsaturated monomer, such as methyl vinyl ether (methoxyethylene) having a molecular weight (M.W.) of about 30,000 to about 1,000,000. These copolymers are available for example as Gantrez AN 139 (M.W. 500,000), AN 119 (M.W. 250,000) and S-97 Pharmaceutical Grade (M.W. 70,000), of GAF Chemicals Corporation.

Other operative polymeric polycarboxylates include the 1:1 copolymers of maleic anhydride with ethyl acrylate, hydroxyethyl methacrylate, N-vinyl-2-pyrrolidone, or ethylene, the latter being available for example as Monsanto EMA No. 1103, M.W. 10,000 and EMA Grade 61, and 1:1 copolymers of acrylic acid with methyl or hydroxyethyl methacrylate, methyl or ethyl acrylate, isobutyl vinyl ether or N-vinyl-2-pyrrolidone. Additional operative polymeric polycarboxylates include copolymers of maleic anhydride with styrene, isobutylene or ethyl vinyl ether; polyacrylic, polyitaconic and polymaleic acids; and sulfoacrylic oligomers of MW as low as 1,000 available as Uniroyal ND-2.

### Surfactants

The present compositions will typically also comprise surfactants, also commonly referred to as sudsing agents. Suitable surfactants are those which are reasonably stable and foam throughout a wide pH range. The surfactant may be anionic, nonionic, amphoteric, zwitterionic, cationic, or mixtures thereof. Preferred surfactants or surfactant mixtures are those that are compatible with the organophosphate agent and other actives in the composition in that the activities of these components are not compromised. Anionic surfactants, such as sodium alkyl sulfate, amphoteric surfactants, such as cocoamidopropyl betaine and their mixtures are typical examples.

Anionic surfactants useful herein include the water-soluble salts of alkyl sulfates having from 8 to 20 carbon atoms in the alkyl radical (e.g., sodium alkyl sulfate) and the water-soluble salts of sulfonated monoglycerides of fatty acids having from 8 to 20 carbon atoms. Sodium lauryl sulfate (SLS) and sodium coconut monoglyceride sulfonates are examples of anionic surfactants of this type. Other suitable anionic surfactants include sarcosinates, isethionates and taurates. Examples for use herein include alkali metal or ammonium salts of these surfactants, such as the sodium and potassium salts of the following: lauroyl sarcosinate, myristoyl sarcosinate, palmitoyl sarcosinate, stearoyl sarcosinate, oleoyl sarcosinate and lauroyl isethionate. Other examples of anionic surfactants are sodium lauryl sulfoacetate, sodium, sodium laureth carboxylate, and sodium dodecyl benzenesulfonate. The composition will typically comprise one or a mixture of anionic surfactants at a level of from about 0.025% to about 9%, from about 0.05% to about 5% or from about 0.1% to about 1%.

Zwitterionic or amphoteric surfactants useful in the present invention include derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate or phosphonate. Suitable betaine surfactants include decyl betaine or 2-(N-decyl-N,N-dimethylammonio)acetate, coco betaine or 2-(N-coco-N, N-dimethyl ammonio)acetate, myristyl betaine, palmityl betaine, lauryl betaine, cetyl betaine, cetyl betaine, stearyl betaine, etc. The amidobetaines are exemplified by cocoamidoethyl betaine, cocamidopropyl betaine (CAPB), and lauramidopropyl betaine.

Cationic surfactants useful in the present invention include derivatives of quaternary ammonium compounds having one long alkyl chain containing from about 8 to 18 carbon atoms such as lauryl trimethylammonium chloride; cetyl pyridinium chloride; cetyl trimethylammonium bromide; coconut alkyltrimethylammonium nitrite; cetyl pyridinium fluoride; etc. Certain cationic surfactants can also function as antimicrobials herein.

Nonionic surfactants include compounds produced by the condensation of alkylene oxide groups (hydrophilic in nature) with an organic hydrophobic compound which may be aliphatic or alkylaromatic in nature. Examples of suitable nonionic surfactants include the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials.

### Abrasives

Dental abrasives may optionally be included in the compositions of the subject invention. Some compositions contemplated herein such as dental gels and finishing gels will preferably be abrasive-free. When present, the abrasive material selected must be one which is compatible with the other components of the composition and will not excessively abrade dentin. Suitable abrasives include, for example, silicas including gels and precipitates, insoluble sodium polymetaphosphate, hydrated alumina, calcium carbonate, dicalcium orthophosphate dihydrate, calcium pyrophosphate, tricalcium phosphate, calcium polymetaphosphate, and resinous abrasive materials such as particulate condensation products of urea and formaldehyde, cross-linked epoxides, and cross-linked polyesters.

Silica dental abrasives of various types are particularly useful because of their unique benefits of exceptional dental cleaning and polishing performance without unduly abrading tooth enamel or dentine. The silica abrasive polishing materials herein, as well as other abrasives, may have an average particle size ranging between about 0.1 to about 30 microns, typically from about 3 to about 20 microns. The abrasive can be precipitated silica or silica gels such as the silica xerogels. Examples include the silica xerogels marketed under the trade name "Syloid" by the W.R. Grace & Company and precipitated silica materials such as those marketed by the J. M. Huber Corporation under the trade name, Zeodent®, particularly the silicas carrying the designation Zeodent® 119, Zeodent® 118, Zeodent® 109 and Zeodent® 129. Mixtures of abrasives can be used such as mixtures of the various grades of Zeodent® silica abrasives listed above. The total amount of abrasive in dentifrice compositions of the subject invention may range from about 6% to about 70% by weight; toothpastes typically contain from about 10% to about 50% of abrasives. Dental solution, mouth spray, mouthwash and non-abrasive gel compositions of the subject invention typically contain little or no abrasive.

### Flavor System

The flavor system is typically added to oral care compositions, to provide a pleasant tasting composition and to effectively mask any unpleasant taste and sensations due to certain components of the composition such as antimicrobial actives or peroxide. Pleasant tasting compositions improve user compliance to prescribed or recommended use of oral care products. The present flavor system will comprise flavor components, in particular those that have been found to be relatively stable in the presence of usual oral care product actives and carriers.

Non-limiting examples of flavor components include flavorants such as peppermint oil, corn mint oil, spearmint oil, oil of wintergreen, clove bud oil, cassia, sage, parsley oil, marjoram, lemon, lime, orange, *cis*-jasmone, 2,5-dimethyl-4-hydroxy-3(2H)-furanone, 5-ethyl-3-hydroxy-4-methyl-2(5H)-furanone, vanillin, ethyl vanillin, anisaldehyde, 3,4-methylenedioxybenzaldehyde, 3,4-dimethoxybenzaldehyde, 4-hydroxybenzaldehyde, 2-methoxybenzaldehyde, benzaldehyde; cinnamaldehyde, hexyl cinnamaldehyde, alpha-methyl cinnamaldehyde, ortho-methoxy cinnamaldehyde, alpha-amyl cinnamaldehydepropenyl guaethol, heliotropine, 4-*cis*-heptenal, diacetyl, methyl-p-tert-butyl phenyl acetate, menthol, methyl salicylate, ethyl salicylate, 1-menthyl acetate, oxanone, alpha-irisone, methyl cinnamate, ethyl cinnamate, butyl cinnamate, ethyl butyrate, ethyl acetate, methyl anthranilate, *iso*-amyl acetate, iso-amyl butyrate, allyl caproate, eugenol, eucalyptol, thymol, cinnamic alcohol, octanol, octanal, decanol, decanal, phenylethyl alcohol, benzyl alcohol, alpha-terpineol, linalool, limonene, citral, maltol, ethyl maltol, anethole, dihydroanethole, carvone, menthone, β-damascenone, ionone, gamma decalactone, gamma nonalactone, gamma undecalactone and mixtures thereof. Generally suitable flavorants are those containing structural features and functional groups that are less prone to redox reactions. Flavor agents or flavorants are generally used in the compositions at levels of from about 0.001% to about 5%, by weight of the composition.

The flavor system will typically include a sweetening agent. Suitable natural water-soluble sweeteners include monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin. Suitable water-soluble artificial sweeteners include soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like. Other suitable sweeteners include dipeptide based sweeteners, such as L-aspartic acid derived sweeteners, including L-aspartyl-L-phenylalanine methyl ester (aspartame) L-alpha-aspartyl-N-(2,2,4,4-tetramethyl-3-thietanyl)-D-alaninamide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclohexylen)-alanine, and the like. Water-soluble sweeteners derived from naturally occurring water-soluble sweeteners, such as a chlorinated derivative of ordinary sugar (sucrose), known under the product description of sucralose and protein based sweeteners such as thaumatoccous danielli (Thaumatin I and II) may be used. Compositions typically contain from about 0.1% to about 10% of sweetener.

Suitable cooling agents or coolants for use in the flavor system include a wide variety of materials such as menthol and its derivatives. Many synthetic coolants are derivatives of or are structurally related to menthol, i.e., containing the cyclohexane moiety, and derivatized with functional groups including carboxamide, ketal, ester, ether and alcohol. Examples include the ρ-menthanecarboxamide compounds such as N-ethyl-ρ-menthan-3-carboxamide, known commercially as "WS-3", and others in the series such as WS-5, WS-12 and WS-14. Examples of menthane carboxy esters include WS-4 and WS-30. An example of a synthetic carboxamide coolant that is structurally unrelated to menthol is N,2,3-trimethyl-2-isopropylbutanamide, known as "WS-23". Additional suitable coolants include 3-1-menthoxypropane-1,2-diol known as TK-10, isopulegol (under the tradename Coolact P) and ρ-menthane-3,8-diol (under the tradename Coolact 38D) all available from Takasago; menthone glycerol acetal known as MGA; menthyl esthers such as menthyl acetate, menthyl acetoacetate, menthyl lactate known as Frescolat® supplied by Haarmann and Reimer, and monomenthyl succinate under the tradename Physcool from V. Mane. Additional useful N-substituted *ρ*-menthane carboxamides are described as having high cooling potency and long lasting sensory effect in WO 2005/049553A1 and include for example, N-(4-cyanomethylphenyl)-*ρ*-menthanecarboxamide, supplied by Givaudan under the designation G-180 coolant.

The flavor system may also include salivating agents, hydration and moisturization agents, and other sensates such as warming agents and numbing agents. These agents are present in the compositions at a level of from about 0.001% to about 10% or from about 0.1% to about 1%. Suitable salivating agents include Jambu® supplied by Takasago and Optaflow^{®} from Symrise. Hydration agents include polyols such as erythritol. Suitable numbing agents include benzocaine, lidocaine, clove bud oil, and ethanol. Warming agents include ethanol, capsicum and nicotinate esters, such as benzyl nicotinate.

### Miscellaneous Carrier Materials

Water employed in the preparation of commercially suitable oral compositions should preferably be of low ion content and free of organic impurities. Water may comprise up to about 99% by weight of the aqueous compositions herein. These amounts of water include the free water which is added plus that which is introduced with other materials, such as with sorbitol.

The present compositions in the form of toothpastes, dentifrices and gels typically will contain some thickening material or binder to provide a desirable consistency. Typical thickening agents include carboxyvinyl polymers, carrageenan, hydroxyethyl cellulose, and water soluble salts of cellulose ethers such as sodium carboxymethylcellulose and sodium hydroxyethyl cellulose. Natural gums such as gum karaya, xanthan gum, gum arabic, and gum tragacanth can also be used. Colloidal magnesium aluminum silicate or finely divided silica can be used as part of the thickening agent to further improve texture. Thickening agents are typically used in an amount from about 0.1% to about 15%, by weight.

The present compositions may also include an alkali metal bicarbonate salt, which may serve a number of functions including abrasive, deodorant, buffering and adjusting pH. Sodium bicarbonate, also known as baking soda, is commonly used. The present composition may contain from about 0.5% to about 30% by weight of an alkali metal bicarbonate salt.

The pH of the present compositions may be adjusted through the use of buffering agents. For example, buffering agents are used to adjust the pH of aqueous compositions such as mouthrinses and dental solutions typically to a range of about pH 4.0 to about pH 8.0. Buffering agents include sodium bicarbonate, monosodium phosphate, trisodium phosphate, sodium hydroxide, sodium carbonate, sodium acid pyrophosphate, citric acid and sodium citrate and maybe included at a level of from about 0.5% to about 10% by weight.

In a specific embodiment, the composition has a pH ranging from 4, 5, 5.5, 6, or 6.5 to 7.5, 8, 9, 10, or 11.

Titanium dioxide may also be added to the present compositions as coloring or opacifying agent typically at a level of from about 0.25% to about 5% by weight.

Poloxamers may be employed in the present compositions. A poloxamer is classified as a nonionic surfactant and may also function as an emulsifying agent, binder, stabilizer, and other related functions. Poloxamers are difunctional block-polymers terminating in primary hydroxyl groups with molecular weights ranging from 1,000 to above 15,000. Poloxamers are sold under the tradename of Pluronics and Pluraflo by BASF including Poloxamer 407 and Pluraflo L4370.

Other emulsifying agents that may be used include polymeric emulsifiers such as the Pemulen® series available from B.F. Goodrich, and which are predominantly high molecular weight polyacrylic acid polymers useful as emulsifiers for hydrophobic substances.

Other optional agents that maybe used include dimethicone copolyols selected from alkyl- and alkoxy-dimethicone copolyols, such as C12 to C20 alkyl dimethicone copolyols and mixtures thereof, as aid in providing positive tooth feel benefits. An example is cetyl dimethicone copolyol marketed under the trade name Abil EM90. The dimethicone copolyol maybe present from about 0.01% to about 25%, typically from about 0.1% to about 5 by weight.

The compositions may optionally contain a humectant, which functions for example to keep toothpaste compositions from hardening upon exposure to air. Certain humectants can also impart desirable sweetness and mouthfeel effects to compositions. Suitable humectants for use herein include glycerin, sorbitol, polyethylene glycol, propylene glycol, and other edible polyhydric alcohols. The humectant may comprise up to about 70%, typically from about 5% to 55%, by weight of the composition.

In some embodiments, the present compositions will comprise a high molecular weight (MW) polyethylene glycol, also called polyethylene oxide (PEO), which provides humectant and mouth moisturization benefits like the more commonly used species of PEO of relatively lower molecular weight (generally from about 200 to about 7000). The high molecular weight PEO's with MW's from about 200,000 to about 7,000,000 have been found to provide excellent mouth moisturization or anti dry mouth benefits as described in commonly assigned U.S. Application 61/257,677. The high MW PEO's provide the anti dry mouth benefit by first lubricating the mouth. This lubrication or lubricity, meaning the lack of friction between elements in contact, provides the opposite effect of dryness. In addition, the high MW PEO's provide actual mouth moisturization by retaining water. Other materials that have been used to treat dry mouth and/or to lubricate the mouth, such as carboxymethylcellulose, for example, do not retain water as well as high MW PEO's. Furthermore, when high MW PEO's are used in combination with polyol humectants (for example, glycerin, erythritol, xylitol, sorbitol, mannitol), a synergistic effect of better moisture retention is achieved, better than either PEO's or polyols used alone, or better than simply an additive effect. It is believed the PEO's are able to deliver superior moisture retention because the high MW PEO's are retained particularly in the soft tissues of the mouth and not easily washed away. By contrast, usual polyol humectants are washed away quickly and perceived to moisturize for less than five minutes. PEO's are retained in the mouth longer and consumer perception of the PEO's moisturization benefit lasts significantly longer than with polyols. In combination with the present organophosphate, it is believed mixed deposition may occur, with the PEO intermingled with the organophosphate particularly on the tooth surface. Or a PEO layer may be deposited on top of the organophosphate layer or vice versa. The combination of PEO and organophosphate has been found to provide improved lubricity and moisturized feel as opposed to a dry mouth feel. The high molecular weight polyethylene oxide may be present for example, in an amount ranging from about 0.001% to about 5.0%, by weight.

### Method of Use

The present invention also relates to methods of use to modify teeth and other oral surfaces to have increased hydrophobic character thereby imparting surface protection, improved tooth health, structure, appearance and textural benefits including erosion protection as well as one or more of caries prevention and control of bacterial activity in the oral cavity which cause undesirable conditions including plaque, calculus, gingivitis, periodontal disease and malodor. The benefits of these compositions may increase over time when the composition is used repeatedly. The method of use or treatment herein may comprise contacting a subject's dental enamel surfaces and mucosa in the mouth with the oral compositions according to the present invention. The method may comprise brushing with a dentifrice or rinsing with a dentifrice slurry or mouthrinse. Other methods include contacting the topical oral gel, denture product, mouthspray, or other form with the subject's teeth and oral mucosa. The subject may be any person or animal whose tooth surface is contacted with the oral composition. By animal is meant to include household pets or other domestic animals, or animals kept in captivity. For example, a method of treatment may include a person brushing a dog's teeth with a dentifrice composition.

The present invention further relates to a method of demonstrating stain-proof efficacy of an oral care composition, comprising the steps:
(a) sticking hydroxyapatite powder uniformly onto a substrate;
(b) immersing the substrate sticked with hydroxyapatite powder into a slurry or solution of the oral care composition;
(c) drying the immersed substrate; and
(d) contacting the dried substrate with a slurry or solution of stain to observe stain's behavior.

In a specific embodiment, step (d) is carried out by dropping a droplet of the slurry or solution of stain onto the dried substrate to observe the droplet's behavior.

In an alternative embodiment, step (d) is carried out by dipping the dried substrate into the slurry or solution of stain for a certain time and then taking out the dipped substrate to observe wetability of the dipped substrate.

The present invention further relates to the use of a substrate coated with hydroxyapatite for demonstrating stain-proof efficacy of an oral care composition.

The present invention further relates to a demonstration product comprising a substrate coated with hydroxyapatite.

The present invention further relates to the use of hydroxyapatite in preparing a demonstration product for demonstrating stain-proof efficacy of an oral care composition.

### EXAMPLES

The following examples further describe and demonstrate embodiments within the scope of the present invention. These examples are given solely for the purpose of illustration and are not to be construed as limitations of the present invention as many variations thereof are possible without departing from the spirit and scope.

Seven examples of the oral care compositions are shown in Table 1. All ingredient amounts are described in weight percentages (wt%) unless otherwise indicated. The oral care compositions are prepared as follows: add water, humectants, part of flavor, colorant, buffer and active to a main mixing tank of 35°C, mixing well and ensuring all the ingredients have dissolved or been well dispersed; add thickening agents and sweetener into the main mixing tank, mixing and homogenizing until well dispersed and homogeneous; add abrasive silica and silica agglomerates, mixing and homogenizing until well dispersed and homogeneous; deaerate; add surfactant solution, rest part of flavor to the main mixing tank, mixing and homogenizing until homogeneous; deaerate; pump out and cool the batch to less than 40°C.

**Table 1**

| **INGREDIENTS** | Ex.1 | Ex. 2 | Ex. 3 | Ex.4 | Ex. 5 | Ex.6 | Ex.7 |
|---|---|---|---|---|---|---|---|
| Sorbitol | 28.3 5 | 28.3 5 | 28.3 5 | 28.35 | 28.3 5 | 28.35 | 28.35 |
| Sodium Carboxymethyl Cellulose | 1 | 1 | 1 | 1 | 1 | 1 | 1 |
| Hydroxyethylcellulose | 0.3 | 0.3 | 0.3 | 0.3 | 0.3 | 0.3 | 0.3 |
| Carrageenan | 0.5 | 0.5 | 0.5 | 0.5 | 0.5 | 0.5 | 0.5 |
| Saccharin Sodium | 0.3 | 0.3 | 0.3 | 0.3 | 0.3 | 0.3 | 0.3 |
| Sodium Fluoride | 0.24 | 0.24 | 0.24 | 0.24 | 0.24 | 0.24 | 0.24 |
| Sodium Citrate Dihydrate | 0.27 | 0.27 | 0.27 | 0.27 | 0.27 | 0.27 | 0.27 |
| Zinc Citrate Dihydrate | 0.79 | 0.79 | 0.79 | 0.79 | 0.79 | 1.866 | 0.062 |
| Abrasive Silica | 17 | 17 | 17 | 17 | 17 | 17 | 17 |
| Sodium Dedeceth-2 Phosphate (MAP) | 0.12 5 | 0.25 | 0.5 | 0.932 5 | 1.25 | 0.932 5 | 0.932 5 |
| Sodium Lauryl Sulfate solution | 2.1 | 2.1 | 2.1 | 2.1 | 2.1 | 2.1 | 2.1 |
| Flavor | 1.2 | 1.2 | 1.2 | 1.2 | 1.2 | 1.2 | 1.2 |
| Colorant | 0.4 | 0.4 | 0.4 | 0.4 | 0.4 | 0.4 | 0.4 |
| Treated Water | 47.4 25 | 47.3 | 47.0 5 | 46.61 75 | 46.3 | 45.54 15 | 47.34 55 |
| Total | 100 | 100 | 100 | 100 | 100 | 100 | 100 |

### Stain-proof demonstration

Hydroxyapatite (HAP) Bio-Gel HTP Gel powder from Bio-Rad Laboratories was used to make HAP substrate for evaluating resistance of stain. Stick the HAP powder uniformly onto a transparent tape. Immerse the HAP tape into the toothpaste slurry for 10 min. Rinse the tape in treated water for 10 seconds, and this step is optional. Dry the tape in ambient for demo use. Several approaches can be used to compare the resistance of stain. One approach is to drop one drop of water, or tea solution or dye solution on the surface of treated HAP tape, and compared the contact angle, maintain the spherical droplet or spread out. Another approach is to lean the tapes to a certain degree angle, then drop liquid onto it see how fast the droplet roll down from the HAP tape. Figs. 1 to 5 show the results.

Unless otherwise indicated, all percentages, ratios, and proportions are calculated based on weight of the total composition. All temperatures are in degrees Celsius (°C) unless otherwise indicated. All measurements made are at 25°C, unless otherwise designated. All component or composition levels are in reference to the active level of that component or composition, and are exclusive of impurities, for example, residual solvents or by-products, which may be present in commercially available sources.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of demonstrating stain-proof efficacy of an oral care composition, comprising the steps:
(a) sticking hydroxyapatite powder uniformly onto a substrate;
(b) immersing the substrate sticked with hydroxyapatite powder into a slurry or solution of the oral care composition;
(c) drying the immersed substrate; and
(d) contacting the dried substrate with a slurry or solution of stain to observe stain's behavior.

2. The method according to claim 1, wherein step (d) is carried out by dropping a droplet of the slurry or solution of stain onto the dried substrate to observe the droplet's behavior.

3. The method according to claim 1, wherein step (d) is carried out by dipping the dried substrate into the slurry or solution of stain for a certain time and then taking out the dipped substrate to observe wetability of the dipped substrate.

4. The use of a substrate coated with hydroxyapatite for demonstrating stain-proof efficacy of an oral care composition.

5. A demonstration product comprising a substrate coated with hydroxyapatite.

6. The use of hydroxyapatite in preparing a demonstration product for demonstrating stain-proof efficacy of an oral care composition.
